# EUROPÄISCHE PATENTSCHRIFT

(11) **EP 3 706 827 B1**
(45) Veröffentlichungstag und Bekanntmachung des Hinweises auf die Patenterteilung: **06.09.2023**
(21) Anmeldenummer: 18800079.8
(22) Anmeldetag: 06.11.2018
(51) Int. Cl.: A61M 5/168, G01F 1/66

(54) **MESSEINRICHTUNG ZUR BESTIMMUNG DES DURCHFLUSSES UND DES VORHANDENSEINS VON FLÜSSIGKEITEN ZUR PARENTERALEN ERNÄHRUNG, DER PHARMAZIE ODER DER BIOTECHNOLOGIE ODER VON BLUT ODER BLUTBESTANDTEILEN ALS MEDIUM**
MEASUREMENT DEVICE TO DETERMINE THE FLOW AND THE PRESENCE OF LIQUIDS FOR PARENTAL FEEDING, FOR PHARMACY OR FOR BIOTECHNOLOGY OR FOR BLOOD OR FOR CONSTITUENTS OF BLOOD, AS MEDIUM
ÉQUIPEMENT DE MESURAGE POUR DÉTERMINER LE FLUX ET LA PRÉSENCE D'UN LIQUIDE POUR ALIMENTATION PARENTÉRALE, POUR LA PHARMACIE OU LA BIOTECHNLOLOGIE OU POUR LE SANG OU POUR LES COMPOSANTS DU SANG, COMME MÉDIUM

(30) Priorität: 09.11.2017 DE 202017106804 U
(43) Veröffentlichungstag der Anmeldung: 16.09.2020
(73) Patentinhaber: Hegewald Medizinprodukte GmbH, 09638 Lichtenberg (DE)
(72) Erfinder: HEGEWALD, Robert, 09638 Lichtenberg (DE)
(74) Vertreter: Kailuweit & Uhlemann Patentanwälte Partnerschaft mbB
(86) Internationale Anmeldenummer: PCT/EP2018/080285
(87) Internationale Veröffentlichungsnummer: WO 2019/091958

(56) Entgegenhaltungen:
- EP-A2- 1 279 368
- DE-A1-102008 002 027
- DE-A1-102008 055 167

## Beschreibung

Die Erfindung betrifft Messeinrichtungen zur Bestimmung des Durchflusses und des Vorhandenseins von Flüssigkeiten zur parenteralen Ernährung, der Pharmazie oder der Biotechnologie oder von Blut oder Blutbestandteilen als Medium mit einem Messrohr, zwei Ultraschallwandlern zur und mit Kopplung an das Messrohr und einem Aufnahmeteil.

Die Druckschrift DE 10 2004 053 673 A1 offenbart eine Vorrichtung zur Bestimmung und/oder Überwachung des Volumen- und/oder Massendurchflusses eines Mediums, das ein Messrohr mit einem Innendurchmesser in einer Strömungsrichtung durchfließt. Mindestens zwei Ultraschallwandler sind so angeordnet, dass die Ultraschallmesssignale entlang definierter Schallpfade aussenden und/oder empfangen. Über eine Regel-/Auswerteeinheit wird der Massedurchfluss des Messmediums im Messrohr anhand der Ultraschallmesssignale nach dem Laufzeitdifferenzprinzip ermittelt. Dazu ist zumindest ein Reflektorelement im Innenraum des Messrohres mit einem definierten Abstand zur Innenwand angeordnet. Angelagerte Partikel am Reflektorelement, beispielsweise als Verschmutzungen im Medium, können das Messergebnis beeinflussen.

Durch die Druckschrift DE 10 2010 053 261 A1 ist eine Messkapsel zur Durchflussmessung bekannt, wobei die Messkapsel eine Anschlussseite mit einer Einlassöffnung und einer Auslassöffnung, ein eine Messstrecke definierendes Messrohr und zwei Ultraschallwandler zur Messung der Durchflussgeschwindigkeit entlang der Messstrecke aufweist. Das Messrohr ist senkrecht zur Anschlusseite angeordnet. Die Messkapsel umfasst weiterhin einen Umlenkraum für das Medium vor dem Messrohr. An diesen ist ein Ultraschallwandler angekoppelt, so dass dessen Ultraschallwellen koaxial in das Messrohr gelangen. Der andere Ultraschallwandler ist so angeordnet, dass die Ultraschallwellen des Messrohres über einen Umlenkspiegel zu diesem Ultraschallwandler gelangen. Nachteilig ist hierbei der nicht verlustfreie Umlenkspiegel. Darüber hinaus beeinflusst dieser die Strömung des Mediums in der Messkapsel. Partikel im Medium können sich anlagern und die Verluste an Ultraschallwellen erhöhen.

Die Druckschrift DE 10 2008 002 027 A1 offenbart eine Messeinrichtung zur Bestimmung des Durchflusses unter Verwendung von Ultraschallwandlern.

Der im Patentanspruch 1 angegebenen Erfindung liegt die Aufgabe zugrunde, den Durchfluss und das Vorhandensein von Flüssigkeiten zur parenteralen Ernährung, der Pharmazie oder der Biotechnologie oder von Blut oder Blutbestandteilen als Medium auch in kleinsten Mengen einfach und sicher zu messen.

Diese Aufgabe wird mit den im Patentanspruch 1 aufgeführten Merkmalen gelöst. Vorteilhafter Ausführungen sind in den Unteransprüchen definiert.

Die Messeinrichtungen zur Bestimmung des Durchflusses und des Vorhandenseins von Flüssigkeiten zur parenteralen Ernährung, der Pharmazie oder der Biotechnologie oder von Blut oder Blutbestandteilen als Medium mit einem Messrohr, zwei Ultraschallwandlern zur und mit Kopplung an das Messrohr und einem Aufnahmeteil zeichnen sich insbesondere dadurch aus, dass der Durchfluss und das Vorhandensein auch kleinster Mengen des Mediums einfach und sicher messbar sind.

Dazu ist das Messrohr ein beidseitig mit jeweils einem Rohrboden geschlossenes Rohr mit an den Endenbereichen angeordneten Mantelflanschen als Anschluss. Das Aufnahmeteil besteht aus zwei miteinander verbundenen und beabstandet zueinander angeordneten Gehäuseteilen, so dass das Messrohr zwischen den Gehäuseteilen platzierbar ist. Die Gehäuseteile weisen jeweils einen Ultraschallwandler auf, wobei wenigstens einer der Ultraschallwandler mittels eines Kolbens verfahrbar ist, so dass die Ultraschallwandler zur Messung an die Rohrböden des Messrohres gekoppelt werden und nach der Messung das Messrohr freigeben. Das Aufnahmeteil weist wenigstens einen Sensor zur Erfassung der Temperatur wenigstens eines Bereichs des Messrohrs und damit des Mediums im Messrohr auf. Weiterhin sind die Ultraschallwandler und der Sensor mit wenigstens einem Datenverarbeitungssystem verbunden.

Die Ultraschallwandler sind vorteilhafterweise jeweils an oder in einem Kolben angeordnet und so mittels eines Bewegungsmechanismus an das Messrohr ankoppelbar und damit lose verbunden. Dazu kann sich wenigstens eine Koppelscheibe und/oder der Boden des Kolbens zwischen einem Ultraschallwandler und dem Rohrboden befinden.

Zur Messung der Strömung im Messrohr ist einer der Ultraschallwandler ein Sender und der andere gegenüber angeordnete Ultraschallwandler ein Empfänger jeweils der Ultraschallwellen. Aus der Ausbreitungsgeschwindigkeit der Ultraschallwellen im fluiden Medium des Messrohres lässt sich die Geschwindigkeit des fluiden Mediums im Messrohr und damit die Durchflussmenge mittels des Datenverarbeitungssystems bestimmen.

Damit ist weiterhin die Voll-Leer-Prüfung des Messrohres oder die Erfassung von Luftblasen verschiedener Größe zur Fehlerminimierung beim Dosiervorgang und/oder zur Prozessüberwachung gegeben.

Der Sensor zur Erfassung der Temperatur kann ein Kaltleiter, ein Heißleiter, ein integrierter Halbleiter-Temperatursensor oder ein als Diode geschalteter Transistor sein. Vorteilhafterweise ist bei Kenntnis der Medientemperatur eine Medienüberprüfung auf Grundlage der Schallgeschwindigkeit des Mediums möglich.

Das Messrohr kann so ein beidseitig mit jeweils einem Rohrboden geschlossenes Rohr mit an den Endenbereichen angeordneten Mantelflanschen zum Anschluss jeweils eines Schlauches sein.

Das Messrohr ist ein steriles oder sterilisierbares Einwegprodukt.

Das Messrohr besteht optional aus zwei Teilen mit jeweils einem Mantelflansch, wobei wenigstens ein Endenbereich des einen Teils in den Endenbereich des anderen Teils eingeschoben ist. Damit lässt sich vorteilhafterweise leicht und ökonomisch günstig ein Messrohr mit einer U-Form herstellen, wobei die Schenkel der U-Form die Mantelflansche sind. Jedes der Teile besitzt dazu einen Mantelflansch.

Wenigstens ein Bereich des Mantels des Messrohres ist eine Ebene. Vorteilhafterweise weist weiterhin das Verbindungsstück des Aufnahmeteils mindestens eine Führungsplatte für die Ebene auf, so dass das Messrohr im Aufnahmeteil fest positioniert ist.

Der Mantelflansch ist optional ein Einschub mit einem Anschlag für einen Endenbereich des Schlauches zum Transport des Mittels zur parenteralen Ernährung oder des Blutes. Die Abmessungen der Bohrung des Mantelflansches sind vorteilhafterweise so ausgebildet, dass der Endenbereich des Schlauches dichtend im Mantelflansch platzierbar ist. Das Messrohr kann aus einem Kunststoff bestehen.

Der Ultraschallwandler befindet sich optional in einem topfförmigen Kolben mit einem einen Boden aufweisenden Hohlzylinder. Der Ultraschallwandler ist auf dem Boden im topfförmigen Kolben oder auf dem Boden auf dem topfförmigen Kolben angeordnet. Damit sind die Scheiben darstellenden Ultraschallwandler einfach handzuhaben. Der topfförmige Kolben mit dem Ultraschallwandler kann so einfach in einer Bohrung des Gehäuseteils platziert werden. Die Anschlüsse des Ultraschallwandlers können einfach aus dem Hohlraum des Kolbens geführt werden. Die nach außen weisende Oberfläche des Bodens kann die Ankoppelfläche des Messrohres sein, wobei jeweils der Rohrboden des Messrohres an den Boden des topfförmigen Kolbens gedrückt wird. Der topfförmige Kolben kann dazu aus einem Metall oder einem Kunststoff bestehen.

Zwischen dem Boden und dem Ultraschallwandler befindet sich optional eine Scheibe aus einem elektrisch nichtleitenden Material und/oder eine elektrisch nichtleitende Kleberschicht. Die Kleberschicht besitzt optional Kugeln aus einem elektrisch nichtleitenden Material. Mittels der Kugeln kann ein bestimmter Abstand zwischen Boden und Ultraschallwandler gewährleistet werden.

Zwischen dem Rohrboden und dem Boden oder dem Ultraschallwandler befindet sich optional wenigstens eine Andruckscheibe. Diese gewährleistet eine weitestgehend verlustfreie Einkopplung der Ultraschallwellen über das Eckstück in das fluide Medium im Messrohr.

Der topfförmige Kolben ist optional in einer Bohrung im Gehäuseteil geführt.

Der verfahrbare Kolben ist optional an einen Exzenter und/oder an eine Schraube in einem Gewindestück und/oder einen Federmechanismus des Gehäuseteils gekoppelt.

Der Ultraschallwandler wird optional mittels eines Federmechanismus auf den Boden des aus einem Kunststoff bestehenden topfförmigen Kolbens gedrückt.

Im Aufnahmeteil befindet sich optional wenigstens eine Einrichtung zur Beeinflussung der Temperatur wenigstens eines Bereichs des Messrohrs und damit des Mediums. Weiterhin ist die Einrichtung zur Beeinflussung der Temperatur mit dem Datenverarbeitungssystem verbunden. Diese kann eine Heizeinrichtung beispielsweise in Form eines elektrischen Widerstands in einem elektrischen Stromkreis oder eine Kühleinrichtung beispielsweise in Form eines Peltierelements sein.

Zur Messung ist einer der Ultraschallwandler ein Sender und der andere gegenüber angeordnete Ultraschallwandler ein Empfänger jeweils der Ultraschallwellen. Weiterhin ist das Datenverarbeitungssystem so mit den Ultraschallwandlern und dem Sensor verbunden, dass aus der Ausbreitungsgeschwindigkeit der Ultraschallwellen im Medium und der Temperatur des Mediums im Messrohr die Durchflussmenge und/oder eine Medienüberprüfung auf Grundlage der Schallgeschwindigkeit des Mediums und/oder eine Voll-Leer-Prüfung und/oder eine Erfassung von Luftblasen zur Fehlerminimierung beim Dosiervorgang und/oder zur Prozessüberwachung bestimmt wird.

Zur Messung ist einer der Ultraschallwandler ein Sender und der andere gegenüber angeordnete Ultraschallwandler ein Empfänger jeweils der Ultraschallwellen. Weiterhin ist das Datenverarbeitungssystem so mit den Ultraschallwandlern, dem Sensor und der Einrichtung zur Beeinflussung der Temperatur verbunden, dass aus der Ausbreitungsgeschwindigkeit der Ultraschallwellen im Medium und der Temperatur des Mediums im Messrohr die Durchflussmenge und/oder eine temperaturabhängige Medienüberprüfung auf Grundlage der temperaturabhängigen Schallgeschwindigkeit des Mediums und/oder eine Voll-Leer-Prüfung und/oder eine Erfassung von Luftblasen zur Fehlerminimierung beim Dosiervorgang und/oder zur Prozessüberwachung bestimmt wird.

Ein Ausführungsbeispiel der Erfindung ist in den Zeichnungen jeweils prinzipiell dargestellt und wird im Folgenden näher beschrieben.

Es zeigen:
- Fig. 1: eine Messeinrichtung zur Bestimmung des Durchflusses von Flüssigkeiten zur parenteralen Ernährung, der Pharmazie oder der Biotechnologie oder von Blut oder Blutbestandteilen,
- Fig. 2: ein Messrohr,
- Fig. 3: ein topfförmiger Kolben mit einem Ultraschallwandler und
- Fig. 4: ein topfförmiger Kolben mit einem aufgeklebten Ultraschallwandler.

Eine Messeinrichtung zur Bestimmung des Durchflusses von Flüssigkeiten zur parenteralen Ernährung, der Pharmazie oder der Biotechnologie oder von Blut oder Butbestandteilen besteht im Wesentlichen aus einem Messrohr 1 und einem Aufnahmeteil 2.

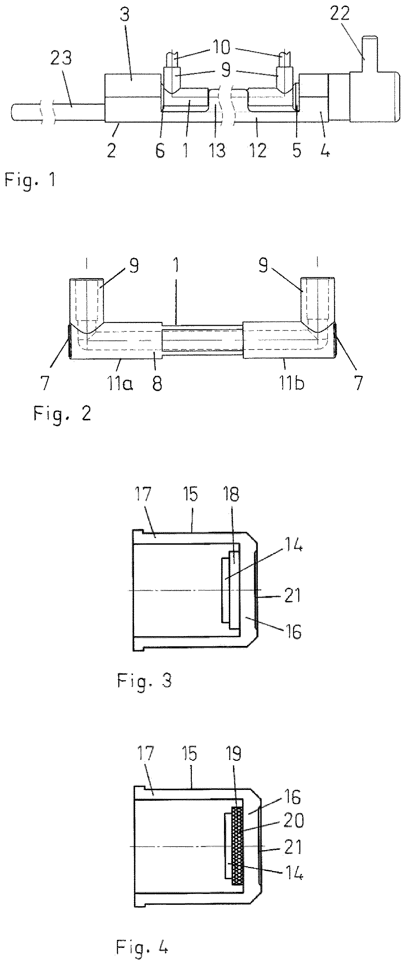

Die Fig. 1 zeigt eine Messeinrichtung zur Bestimmung des Durchflusses von Flüssigkeiten zur parenteralen Ernährung, der Pharmazie oder der Biotechnologie oder von Blut oder Blutbestandteilen in einer prinzipiellen Darstellung.

Das Aufnahmeteil 2 besteht aus zwei miteinander verbundenen und beabstandet zueinander angeordneten Gehäuseteilen 3, 4 besteht. Das Messrohr 1 ist zwischen den Gehäuseteilen 3, 4 platzierbar. Die Gehäuseteile 3, 4 weisen jeweils einen Ultraschallwandler 14 auf, wobei wenigstens einer der Ultraschallwandler 14 mittels eines Kolbens 5 im Gehäuseteil 4 verfahrbar ist. Der andere gegenüber dem bewegbaren Ultraschallwandler 14 angeordnete Ultraschallwandler 14 befindet sich in einem feststehenden Kolben 6 im anderen Gehäuseteil 4. Damit werden die Ultraschallwandler 14 zur Messung an die Rohrböden 7 des Messrohres 1 gekoppelt und nach der Messung ist das Messrohr 1 freigegeben, so dass das Messrohr 1 aus dem Aufnahmeteil 2 entfernt werden kann. Das Aufnahmeteil 2 weist wenigstens einen Sensor zur Erfassung der Temperatur wenigstens eines Bereichs des Messrohrs 1 und damit des Mediums im Messrohr 1 auf. Dieser und die Ultraschallwandler 14 sind mit wenigstens einem Datenverarbeitungssystem verbunden. Das Aufnahmeteil 2 kann aus einem Metall oder einem Kunststoff bestehen.

Die Fig. 2 zeigt ein Messrohr 1 in einer prinzipiellen Darstellung.

Das Messrohr 1 ist ein beidseitig mit jeweils einem Rohrboden 7 geschlossenes Rohr 8 mit an den Endenbereichen angeordneten Mantelflanschen 9 zum Anschluss jeweils eines Schlauches 10. Das Messrohr 1 ist ein steriles Einwegprodukt und besteht aus einem Kunststoff. Das Messrohr 1 besteht weiterhin aus zwei Teilen 11a, 11b mit jeweils einem Mantelflansch 9, wobei wenigstens ein Endenbereich des einen Teils 11a in den Endenbereich des anderen Teils 11b eingeschoben ist. Der Mantelflansch 9 ist ein Einschub mit einem Anschlag für einen Endenbereich des Schlauches 10 zum Transport des Mittels zur parenteralen Ernährung oder des Blutes.

Wenigstens ein Bereich des Mantels des Messrohres 1 ist eine Ebene. Das die Gehäuseteile 3, 4 miteinander verbindende Verbindungsstück 12 des Aufnahmeteils 1 weist mindestens eine Führungsplatte 13 für die Ebene auf, so dass das Messrohr 1 im Aufnahmeteil2 fest und damit insbesondere verdrehsicher positioniert ist.

Die Fig. 3 zeigt einen topfförmigen Kolben 5, 6 mit einem Ultraschallwandler 14 in einer prinzipiellen Darstellung.

Der Ultraschallwandler 14 befindet sich in einem topfförmigen Kolben 15 mit einem einen Boden 16 aufweisenden Hohlzylinder 17. Dazu ist der Ultraschallwandler 14 auf dem Boden 16 im topfförmigen Kolben 15 angeordnet. Zwischen dem Boden 16 und dem Ultraschallwandler 14 befindet sich eine Scheibe 18 aus einem elektrisch nichtleitenden Material.

Die Fig. 4 zeigt einen topfförmigen Kolben 15 mit einem aufgeklebten Ultraschallwandler 14 in einer prinzipiellen Darstellung.

In einer Ausführungsform des Kolbens 15 befindet sich zwischen Ultraschallwandler 14 und Boden 16 eine elektrisch nichtleitende Kleberschicht 19, die Kugeln 20 aus einem elektrisch nichtleitenden Material besitzt.

Die nach außen weisende Oberfläche 21 des Bodens 16 der topfförmigen Kolben 15 der Ausführungsformen ist die Ankoppelfläche des Messrohres 1, wobei jeweils der Rohrboden 7 des Messrohres 1 an den Boden 16 des jeweiligen topfförmigen Kolbens 15 gedrückt wird.

Der topfförmige Kolben 15 kann vorteilhafterweise in einer Bohrung im jeweiligen Gehäuseteil 3, 4 geführt sein. Der verfahrbare Kolben 15 kann dazu an einen Exzenter und/oder an eine Schraube in einem Gewindestück und/oder einen Federmechanismus des Gehäuseteils 4 gekoppelt sein. Zur Bewegung besitzt dieses einen Griff 22. Der topfförmige Kolben 15 kann aus einem Metall oder einem Kunststoff bestehen.

Zur Messung der Strömung im Messrohr 1 ist einer der Ultraschallwandler 14 ein Sender und der andere gegenüber angeordnete Ultraschallwandler 14 ein Empfänger jeweils der Ultraschallwellen. Das Datenverarbeitungssystem ist so mit den Ultraschallwandlern 14 verbunden, dass aus der Ausbreitungsgeschwindigkeit der Ultraschallwellen im Mittel zur parenteralen Ernährung oder im Blut des Messrohres die Geschwindigkeit des Mittels zur parenteralen Ernährung oder des Blutes im Messrohr 1 und damit die Durchflussmenge bestimmt wird. Dazu sind entsprechende Kabel 23 zur Energieversorgung und als Datenleitung vorhanden.

### Bezugszeichen

- 1: Messrohr
- 2: Aufnahmeteil
- 3: Gehäuseteil
- 4: Gehäuseteil
- 5: verfahrbarer Kolben
- 6: feststehender Kolben
- 7: Rohrboden
- 8: Rohr
- 9: Mantelflansch
- 10: Schlauch
- 11: Teile des Messrohres
- 12: Verbindungsstück
- 13: Führungsplatte
- 14: Ultraschallwandler
- 15: Kolben
- 16: Boden
- 17: Hohlzylinder
- 18: Scheibe
- 19: Kleberschicht
- 20: Kugel
- 21: Oberfläche
- 22: Griff
- 23: Kabel

## Patentansprüche

1. Messeinrichtung zur Bestimmung des Durchflusses und des Vorhandenseins von Flüssigkeiten zur parenteralen Ernährung, der Pharmazie oder der Biotechnologie oder von Blut oder Blutbestandteilen als Medium
• mit einem Messrohr (1),
• zwei Ultraschallwandlern (14) zur und mit Kopplung an das Messrohr (1) und
• einem Aufnahmeteil (2), wobei
• das Messrohr (1) mit einer U-Form ein beidseitig mit jeweils einem Rohrboden (7) geschlossenes Rohr (8) mit an den Endenbereichen angeordneten Mantelflanschen (9) als Anschluss ist, wobei Schenkel der U-Form die Mantelflansche (9) sind und wenigstens ein Bereich des Mantels des Messrohres (1) eine Ebene ist,
• wobei das Messrohr (1) ein steriles oder sterilisierbares Einwegprodukt ist und
• dass das Aufnahmeteil (2) aus zwei miteinander verbundenen und beabstandet zueinander angeordneten Gehäuseteilen (3, 4) besteht, so dass das Messrohr (1) zwischen den Gehäuseteilen (3, 4) platzierbar ist,
• dass die Gehäuseteile (3, 4) jeweils einen Ultraschallwandler (14) aufweisen,
• wobei wenigstens einer der Ultraschallwandler (14) mittels eines Kolbens (5) verfahrbar ist, so dass die Ultraschallwandler (14) zur Messung an die Rohrböden (7) des Messrohres (1) gekoppelt werden und nach der Messung das Messrohr (1) freigegeben ist,
• dass das Aufnahmeteil (2) wenigstens einen Sensor zur Erfassung der Temperatur wenigstens eines Bereichs des Messrohrs (1) und damit des Mediums im Messrohr (1) aufweist, und dass die Ultraschallwandler (14) und der Sensor mit wenigstens einem Datenverarbeitungssystem verbunden sind,
• wobei zur Messung einer der Ultraschallwandler (14) ein Sender und der andere gegenüber angeordnete Ultraschallwandler (14) ein Empfänger jeweils der Ultraschallwellen ist und dass das Datenverarbeitungssystem so mit den Ultraschallwandlern (14) und dem Sensor verbunden ist, dass aus der Ausbreitungsgeschwindigkeit der Ultraschallwellen im Medium und der Temperatur des Mediums im Messrohr (1) die Durchflussmenge und/oder eine Medienüberprüfung auf Grundlage der Schallgeschwindigkeit des Mediums und/oder eine Voll-Leer-Prüfung und/oder eine Erfassung von Luftblasen zur Fehlerminimierung beim Dosiervorgang und/oder zur Prozessüberwachung bestimmt wird.

2. Messeinrichtung nach Patentanspruch 1, **dadurch gekennzeichnet, dass** das Messrohr (1) aus zwei Teilen (11) mit jeweils einem Mantelflansch (9) besteht, wobei wenigstens ein Endenbereich des einen Teils (11a) in den Endenbereich des anderen Teils (11b) eingeschoben ist.

3. Messeinrichtung nach Patentanspruch 1, **dadurch gekennzeichnet, dass** wenigstens ein Bereich des Mantels des Messrohres (1) eine Ebene ist und dass das Verbindungsstück (12) des Aufnahmeteils (2) mindestens eine Führungsplatte (13) für die Ebene aufweist, so dass das Messrohr (1) im Aufnahmeteil (2) fest positioniert ist.

4. Messeinrichtung nach Patentanspruch 1, **dadurch gekennzeichnet, dass** der Mantelflansch (9) ein Einschub mit einem Anschlag für einen Endenbereich des Schlauches (10) zum Transport des Mittels zur parenteralen Ernährung oder des Blutes ist.

5. Messeinrichtung nach Patentanspruch 1, **dadurch gekennzeichnet, dass** sich der Ultraschallwandler (14) in einem topfförmigen Kolben (5, 6) mit einem einen Boden (16) aufweisenden Hohlzylinder (17) befindet und dass der Ultraschallwandler (14) auf dem Boden (16) im topfförmigen Kolben (5, 6) oder auf dem topfförmigen Kolben (5, 6) angeordnet ist.

6. Messeinrichtung nach Patentanspruch 5,
**dadurch gekennzeichnet, dass** sich zwischen dem Boden (16) und dem Ultraschallwandler (14) eine Scheibe (18) aus einem elektrisch nichtleitenden Material und/oder eine elektrisch nichtleitende Kleberschicht (19) befindet.

7. Messeinrichtung nach Patentanspruch 6,
**dadurch gekennzeichnet, dass** die Kleberschicht (19) Kugeln (20) aus einem elektrisch nichtleitenden Material besitzt.

8. Messeinrichtung nach den Patentansprüchen 1 und 5,
**dadurch gekennzeichnet, dass** sich zwischen dem Rohrboden (7) und dem Boden (16) oder dem Ultraschallwandler (14) wenigstens eine Andruckscheibe befindet.

9. Messeinrichtung nach den Patentansprüchen 1 und 5,
**dadurch gekennzeichnet, dass** der topfförmige Kolben (5, 6) in einer Bohrung im Gehäuseteil (3, 4) geführt ist.

10. Messeinrichtung nach Patentanspruch 1, **dadurch gekennzeichnet, dass** der verfahrbare Kolben (5) an einen Exzenter und/oder an eine Schraube in einem Gewindestück und/oder einen Federmechanismus des Gehäuseteils (4) gekoppelt ist.

11. Messeinrichtung nach Patentanspruch 5,
**dadurch gekennzeichnet, dass** der Ultraschallwandler (14) mittels eines Federmechanismus auf den Boden (16) des aus einem Kunststoff bestehenden topfförmigen Kolbens (5, 6) gedrückt wird.

12. Messeinrichtung nach Patentanspruch 1, **dadurch gekennzeichnet, dass** sich im Aufnahmeteil (2) wenigstens eine Einrichtung zur Beeinflussung der Temperatur wenigstens eines Bereichs des Messrohrs (1) und damit des Mediums befindet und die Einrichtung zur Beeinflussung der Temperatur mit dem Datenverarbeitungssystem verbunden ist.

13. Messeinrichtung nach Patentansprüchen 1 und 12, **dadurch gekennzeichnet, dass** zur Messung einer der Ultraschallwandler (14) ein Sender und der andere gegenüber angeordnete Ultraschallwandler (14) ein Empfänger jeweils der Ultraschallwellen ist und dass das Datenverarbeitungssystem so mit den Ultraschallwandlern (14), dem Sensor und der Einrichtung zur Beeinflussung der Temperatur verbunden ist, dass aus der Ausbreitungsgeschwindigkeit der Ultraschallwellen im Medium und der Temperatur des Mediums im Messrohr (1) die Durchflussmenge und/oder eine temperaturabhängige Medienüberprüfung auf Grundlage der temperaturabhängigen Schallgeschwindigkeit des Mediums und/oder eine Voll-Leer-Prüfung und/oder eine Erfassung von Luftblasen zur Fehlerminimierung beim Dosiervorgang und/oder zur Prozessüberwachung bestimmt wird.

## Claims

1. Measuring device for determining the flow rate and the presence of fluids for parenteral feeding, pharmaceutics or biotechnology or of blood or blood components as the medium,
• comprising a measuring tube (1),
• two ultrasonic transducers (14) for and having coupling to the measuring tube (1) and
• a receiving part (2), wherein
• the measuring tube (1) is a tube (8) which is U-shaped, is closed on both ends by a tube plate (7), and has shell flanges (9) arranged on the end regions as a connection, wherein limbs of the U-shape are the shell flanges (9) and at least one region of the shell of the measuring tube (1) is a plane,
• wherein the measuring tube (1) is a sterile or sterilizable disposable product, and
• in that the receiving part (2) consists of two interconnected and spaced- apart housing parts (3, 4) such that the measuring tube (1) can be placed between the housing parts (3, 4),
• in that the housing parts (3, 4) each have an ultrasonic transducer (14),
• wherein at least one of the ultrasonic transducers (14) can be moved by means of a piston (5) such that, for measuring, the ultrasonic transducers (14) are coupled to the tube plates (7) of the measuring tube (1) and, after measuring, the measuring tube (1) is released,
• in that the receiving part (2) has at least one sensor for detecting the temperature of at least one region of the measuring tube (1) and thus of the medium in the measuring tube (1), and in that the ultrasonic transducers (14) and the sensor are connected to at least one data processing system,
• wherein, for measuring, one of the ultrasonic transducers (14) is a transmitter and the other oppositely arranged ultrasonic transducer (14) is a receiver of the ultrasonic waves in each case, and in that the data processing system is connected to the ultrasonic transducers (14) and the sensor such that the volumetric flow rate and/or a media check on the basis of the acoustic velocity of the medium and/or a full-empty check and/or detection of air bubbles for minimizing errors during the metering process and/or for process monitoring is determined from the propagation rate of the ultrasonic waves in the medium and the temperature of the medium in the measuring tube (1).

2. Measuring device according to claim 1, **characterized in that** the measuring tube (1) consists of two parts (11) each having an shell flange (9), at least one end region of one part (11a) being inserted into the end region of the other part (11b).

3. Measuring device according to claim 1, **characterized in that** at least one region of the shell of the measuring tube (1) is a plane and **in that** the connecting piece (12) of the receiving part (2) has at least one guide plate (13) for the plane such that the measuring tube (1) is fixedly positioned in the receiving part (2).

4. Measuring device according to claim 1, **characterized in that** the shell flange (9) is an insert having a stop for an end region of the tubing (10) for transporting the means for parenteral feeding or the blood.

5. Measuring device according to claim 1, **characterized in that** the ultrasonic transducer (14) is located in a pot-shaped piston (5, 6) having a hollow cylinder (17) that has a base (16) and **in that** the ultrasonic transducer (14) is arranged on the base (16) in the pot-shaped piston (5, 6) or on the pot-shaped piston (5, 6).

6. Measuring device according to claim 5,
**characterized in that** a disk (18) made of an electrically non-conductive material and/or an electrically non-conductive adhesive layer (19) is located between the base (16) and the ultrasonic transducer (14).

7. Measuring device according to claim 6,
**characterized in that** the adhesive layer (19) comprises spheres (20) made of an electrically non-conductive material.

8. Measuring device according to claims 1 and 5,
**characterized in that** at least one pressing disk is located between the tube plate (7) and the base (16) or the ultrasonic transducer (14).

9. Measuring device according to claims 1 and 5,
**characterized in that** the pot-shaped piston (5, 6) is guided in a bore in the housing part (3, 4).

10. Measuring device according to claim 1, **characterized in that** the movable piston (5) is coupled to an eccentric and/or to a screw in a threaded piece and/or a spring mechanism of the housing part (4).

11. Measuring device according to claim 5,
**characterized in that** the ultrasonic transducer (14) is pressed by means of a spring mechanism onto the base (16) of the pot-shaped piston (5, 6) that consists of a plastics material.

12. Measuring device according to claim 1, **characterized in that** at least one device for influencing the temperature of at least one region of the measuring tube (1) and thus of the medium is located in the receiving part (2) and the device for influencing the temperature is connected to the data processing system.

13. Measuring device according to claims 1 and 12, **characterized in that,** for measuring, one of the ultrasonic transducers (14) is a transmitter and the other oppositely arranged ultrasonic transducer (14) is a receiver of the ultrasonic waves in each case, and **in that** the data processing system is connected to the ultrasonic transducers (14), the sensor and the device for influencing the temperature such that the volumetric flow rate and/or a temperature-dependent media check on the basis of the temperature-dependent acoustic velocity of the medium and/or a full-empty check and/or detection of air bubbles for minimizing errors during the metering process and/or for process monitoring is determined from the propagation rate of the ultrasonic waves in the medium and the temperature of the medium in the measuring tube (1).

## Revendications

1. Dispositif de mesure permettant de déterminer le débit et la présence de liquides destinés à la nutrition parentérale, au domaine de la pharmacie ou de la biotechnologie, ou de sang ou de composants sanguins en tant que milieu
• comportant un tube de mesure (1),
• deux transducteurs ultrasoniques (14) pour le couplage au tube de mesure (1) et couplés à celui-ci, et
• une partie de réception (2), dans lequel
• le tube de mesure (1) comportant une forme en U est un tube (8) fermé des deux côtés par respectivement un fond de tube (7) et comportant des brides d'enveloppe (9) disposées au niveau des zones d'extrémités et servant de raccordement, dans lequel des branches de la forme en U sont les brides d'enveloppe (9) et au moins une zone de l'enveloppe du tube de mesure (1) est un plan,
• dans lequel le tube de mesure (1) est un produit jetable stérile ou stérilisable et
• **en ce que** la partie de réception (2) est constituée de deux parties de boîtier (3, 4) reliées l'une à l'autre et disposées à distance l'une de l'autre, de telle sorte que le tube de mesure (1) peut être placé entre les parties de boîtier (3, 4),
• **en ce que** les parties de boîtier (3, 4) présentent respectivement un transducteur ultrasonique (14),
• dans lequel au moins l'un des transducteurs ultrasoniques (14) peut être déplacé à l'aide d'un piston (5), de telle sorte que les transducteurs ultrasoniques (14) sont accouplés aux fonds de tube (7) du tube de mesure (1) pour la mesure, et le tube de mesure (1) est libéré après la mesure,
• **en ce que** la partie de réception (2) présente au moins un capteur destiné à détecter la température d'au moins une zone du tube de mesure (1), et donc du milieu dans le tube de mesure (1), **et en ce que** les transducteurs ultrasoniques (14) et le capteur sont reliés à au moins un système de traitement de données,
• dans lequel, pour la mesure, l'un des transducteurs ultrasoniques (14) est un émetteur et l'autre transducteur ultrasonique (14) disposé en face est un récepteur, respectivement des ondes ultrasonores, **et en ce que** le système de traitement de données est relié aux transducteurs ultrasoniques (14) et au capteur de telle sorte que, à partir de la vitesse de propagation des ondes ultrasonores dans le milieu et de la température du milieu dans le tube de mesure (1), on détermine le débit et/ou une analyse du milieu en fonction de la vitesse sonique du milieu et/ou un test plein-vide et/ou une détection de bulles d'air pour minimiser les erreurs lors de l'opération de dosage et/ou pour surveiller le processus.

2. Dispositif de mesure selon la revendication 1, **caractérisé en ce que** le tube de mesure (1) est constitué de deux parties (11) comportant respectivement une bride d'enveloppe (9), dans lequel au moins une zone d'extrémité d'une partie (11a) est insérée dans la zone d'extrémité de l'autre partie (11b).

3. Dispositif de mesure selon la revendication 1, **caractérisé en ce qu'**au moins une zone de l'enveloppe du tube de mesure (1) est un plan, **et en ce que** la pièce de liaison (12) de la partie de réception (2) présente au moins une plaque de guidage (13) pour le plan, de telle sorte que le tube de mesure (1) est positionné fixement dans la partie de réception (2).

4. Dispositif de mesure selon la revendication 1, **caractérisé en ce que** la bride d'enveloppe (9) est un insert comportant une butée pour une zone d'extrémité du tuyau (10) pour le transport du produit destiné à la nutrition parentérale ou du sang.

5. Dispositif de mesure selon la revendication 1, **caractérisé en ce que** le transducteur ultrasonique (14) se trouve dans un piston (5, 6) en forme de pot comportant un cylindre creux (17) présentant un fond (16), **et en ce que** le transducteur ultrasonique (14) est disposé sur le fond (16) dans le piston (5, 6) en forme de pot ou est disposé sur le piston (5, 6) en forme de pot.

6. Dispositif de mesure selon la revendication 5,
**caractérisé en ce qu'**un disque (18) fabriqué à partir d'un matériau électriquement non conducteur et/ou d'une couche adhésive (19) électriquement non conductrice se trouve entre le fond (16) et le transducteur ultrasonique (14).

7. Dispositif de mesure selon la revendication 6,
**caractérisé en ce que** la couche adhésive (19) présente des boules (20) fabriquées à partir d'un matériau électriquement non conducteur.

8. Dispositif de mesure selon les revendications 1 et 5,
**caractérisé en ce qu'au** moins un disque de pression se trouve entre le fond de tube (7) et le fond (16) ou le transducteur ultrasonique (14).

9. Dispositif de mesure selon les revendications 1 et 5,
**caractérisé en ce que** le piston (5, 6) en forme de pot est guidé dans un alésage dans la partie de boîtier (3, 4).

10. Dispositif de mesure selon la revendication 1, **caractérisé en ce que** le piston (5) déplaçable est accouplé à un excentrique et/ou à une vis dans une pièce filetée et/ou à un mécanisme à ressort de la partie de boîtier (4).

11. Dispositif de mesure selon la revendication 5,
**caractérisé en ce que** le transducteur ultrasonique (14) est pressé, à l'aide d'un mécanisme à ressort, sur le fond (16) du piston (5, 6) en forme de pot constitué d'une matière plastique.

12. Dispositif de mesure selon la revendication 1, **caractérisé en ce qu'**au moins un dispositif permettant d'influer sur la température d'au moins une zone du tube de mesure (1), et donc du milieu, se trouve dans la partie de réception (2), et le dispositif permettant d'influer sur la température est relié au système de traitement de données.

13. Dispositif de mesure selon les revendications 1 et 12, **caractérisé en ce que,** pour la mesure, l'un des transducteurs ultrasoniques (14) est un émetteur et l'autre transducteur ultrasonique (14) disposé en face est un récepteur, respectivement des ondes ultrasonores, **et en ce que** le système de traitement de données est relié aux transducteurs ultrasoniques (14), au capteur et au dispositif permettant d'influer sur la température, de telle sorte que, à partir de la vitesse de propagation des ondes ultrasonores dans le milieu et de la température du milieu dans le tube de mesure (1), on détermine le débit et/ou une analyse du milieu dépendant de la température en fonction de la vitesse sonique dépendante de la température, du milieu et/ou un test plein-vide et/ou une détection de bulles d'air pour minimiser les erreurs lors de l'opération de dosage et/ou pour surveiller le processus.
